# EUROPEAN PATENT APPLICATION

(11) **EP 0 792 660 A2**
(43) Date of publication of application: **03.09.1997**
(21) Application number: 97301192.7
(22) Date of filing: 24.02.1997
(51) Int. Cl.: A61M 25/06

(54) **Introducer system**

(30) Priority: 29.02.1996 US 608919
(71) Applicant: MEDTRONIC, INC., Minneapolis, Minnesota 55432 (US)
(72) Inventor: Gammage, Michael D., Moseley, Birmingham B1 39 (GB); Lahtinen, Stuart P., Minneapolis, Minnesota 55419 (GB); Pohndorf, Peter J., Stillwater, Minnesota 55082 (US)
(74) Representative: Hughes, Andrea Michelle

(57) **Abstract**

An introducer system for a lead, catheter, probe or the like, employing a dilator 42 and introducer sheath 40 assembly for dilating a body vessel in preparation for introducing the lead, etc., through the lumen 58 of the introducer sheath that is easily advanced into the body vessel while minimizing injury to the vessel wall. The elongated tubular dilator 42 is formed with a tapered distal end portion extending proximally of a reduced diameter distal tip end to a relatively uniform major diameter. The introducer sheath major lumen diameter is dimensioned to fit snugly over the major diameter and to have a reduced distal end opening diameter from the major lumen diameter. An annular recess 70 is formed in the dilator adjacent to the distal end portion for receiving the reduced distal end opening diameter, such that when so assembled, a smooth transition of the dilator and sheath outer surfaces are presented proximal to the tapered distal end portion. The introducer sheath wall thickness may also be tapered in a distal introducer sheath portion. The annular recess and/or the sheath distal end opening are shaped to provide a cam surface to allow retraction of the distal end portion of the dilator past the reduced diameter sheath distal end opening. The sheath preferably is constructed to readily tear in a longitudinal direction and thus permits the system to be removed from the venous system without having to withdraw the sheath over an end of the lead, etc.

## Description

This invention relates generally to an introducer system for a lead or catheter employing a dilator and introducer sheath assembly for dilating a body vessel in preparation for introducing the lead or catheter through the lumen of the introducer sheath that is easily advanced into the body vessel while minimizing injury to the vessel wall.

A multi-step procedure is often used to introduce electrical stimulation leads, catheters, probes or the like into a body vessel, most notably into the vascular system, typically the venous system. Generally this procedure consists of inserting a hollow needle into a blood vessel, such as the subclavian vein. A wire guide is then passed through the needle into the interior portion of the vessel. The needle is then withdrawn and an introducer sheath and dilator assembly is then inserted over the wire guide into the vessel. The assembly is advanced into a suitable position within the vessel, i.e. so that the distal end is well within the vessel but the proximal end is outside the patient. Next, the dilator and wire guide are removed from the introducer sheath lumen. The introducer sheath is left in position and therefore offers direct access from outside the patient into the blood vessel lumen. Then, a lead, catheter or the like can be passed through the introducer sheath lumen into the blood vessel lumen and ultimately be positioned in a desired site, e.g., within the patient's selected heart chamber or an associated blood vessel. Such a system and method is commonly employed for percutaneously introducing pacing leads for permanent or temporary pacemakers or arrhythmia control devices. In the implantable pacemaker context, an implantable pulse generator is electrically connected to the heart by at least one pacing lead, e.g. an endocardial lead introduced transvenously. More specifically, an endocardial lead provides an electrical pathway between the pacemaker pulse generator, connected to the proximal end of the lead, and endocardial tissue, in contact with one or more electrodes at the distal end of the lead. Endocardial tissue refers to a specific layer of tissue in the interior of the heart's chambers. In such a manner, electrical pacing pulses emitted by the pacemaker travel through the endocardial lead conductor and depolarize the endocardial tissue of the heart in contact with the electrode(s). The contraction of the heart is effected by the propagation of the evoked depolarization.

Endocardial pacing leads are often placed in contact with the endocardial tissue by passage through a venous access, such as the subclavian vein or one of its tributaries. In such a manner, transvenous endocardial leads offer as an advantage that they may be placed into contact with the heart without requiring major thoracic surgery. Rather, once the transvenous endocardial leads are introduced into a vein and manoeuvered therefrom into contact with the heart, the vein access may be closed and the lead connector end may be tunnelled under the skin to the implant site of the pulse generator and attached thereto.

Such pacemaker leads typically have a relatively bulky connector pin assembly at the proximal end, and the introducer sheath is removed from the lead body at the end of the procedure by being split apart. In such a manner, the introducer sheath does not have to be removed over the relatively bulky connector pin assembly at the proximal end of the lead body.

One purpose of this well known procedure is to expand the lumen of the blood vessel accessed at the puncture site so that it can receive the distal end of the lead. The distal tip of the dilator is tapered to expand the blood vessel lumen as it is advanced through it. After the dilator is withdrawn, the sheath maintains the expansion of the blood vessel lumen and provides an introducer lumen for the distal end and body of the lead.

A problem exists in pacemaker patients who have had multiple leads implanted over time. Scar tissue at the site of lead introduction into a blood vessel during implantation has been found to create difficulties with past lead introduction systems. Specifically the relatively tough scar tissue hinders the introduction of a dilator and introducer sheath assembly. Many times, only through use of larger incisions than are otherwise desirable is such an assembly able to be inserted and advanced.

To provide such access under the best of circumstances, the introducer sheath must be flexible in order to permit the introducer sheath to bend and conform with blood vessel curves and bends. After placement in the vessel lumen, the introducer sheath end is substantially parallel to the blood vessel lumen, and a lead which is introduced therethrough is properly aligned with the vessel lumen. If the sheath did not conform to the vessel shape, a lead introduced through its distal end opening would abut against the vessel wall, possibly injuring the vessel wall and damaging the lead.

In view of these considerations, the sheath is formed with a thin walled, typically splittable, cylindrical tube that has a typically uniform, discrete wall thickness and a cylindrical sheath lumen. The dilator is typically also cylindrical and sized to fit within the sheath lumen. In the introduction process for introducing the sheath and dilator assembly, the distal end of the thin walled sheath tube is located proximal to or adjacent to the taper of the distal end of the dilator. At the junction of the sheath distal end and the dilator, an annular step or bulge is present due to the wall thickness of the introducer sheath. During the introduction the assembly through the blood vessel curves and bends, the annular step or bulge can catch on the vessel wall which can either impede the forward extension of the assembly over the wire, directly injure the vessel wall or mis-align the distal end opening of the sheath with respect to the vessel lumen such that the lead distal tip may injure the vessel wall when it is advanced distally of the sheath distal end opening.

One approach to minimizing this problem has been to ensure that there is virtually no gap or play between the inner lumen of the sheath and the outer diameter of the dilator. In Furukawa U.S. Patent No. 4,850,975, the dilator outer diameter and the sheath lumen are dimensioned with an interference fit that is accommodated by a slit in the dilator wall to allow its outer diameter to be compressed within the introducer sheath. However, the sheath distal end continues to present an annular step.

Another approach has been to form the distal end portion of the introducer sheath with a tapered wall width decreasing from the nominal or major sheath wall thickness to a minimum wall thickness at the sheath distal end opening. Such an introducer sheath, exhibiting a relatively straight taper when assembled over a dilator, is provided by the assignee of the present application and designated as the SOLO-TRAC® introducer sheath. A further sheath of the assignee of the present application designated the QUICKSPLIT ® introducer sheath exhibits a more rounded curvature of the tapered distal portion when assembled over a dilator.

In another approach, the introducer sheath may be formed with a tapered distal tip portion having an inside diameter that is necked down slightly from the nominal sheath lumen so that the necked down end fits snugly against the outer diameter and surface of the dilator.

Although these approaches tend to make introduction of the assembled sheath and dilator easier and to reduce the potential for injury, further improvement is desirable. While these approaches are an improvement over the blunt ended sheaths of the prior art, the residual step enlargement at the sheath-dilator transition zone can still catch on blood vessel walls or scar tissue and impede advancement of the dilator/sheath assembly into a blood vessel and/or damage the blood vessel wall.

It is therefore an object of the present invention to provide an introducer system having a sheath and dilator formed to minimize catching on blood vessel walls during introduction and advancement in the vessel.

It is a further object of the invention to provide an introducer system having a sheath and dilator assembly providing a smooth transition at the junction of the sheath distal end with the dilator outer diameter.

These objects are met by the present invention which, in one aspect, provides an introducer system for a lead, catheter, probe or the like, employing a dilator and introducer sheath assembly for dilating a body vessel in preparation for introducing the lead, etc., through the lumen of the introducer sheath that is easily advanced into the body vessel while minimizing injury to the vessel wall.

According to one aspect of the present invention, there is provided an introducer system for dilating a body vessel or the like for introducing an introducer sheath into the vessel lumen comprising:
elongate tubular dilator means for dilating a body vessel lumen having a distal tip end and a dilator outer surface;
elongate introducer sheath means body enclosing a sheath lumen therein and extending between openings in a sheath distal end and a sheath proximal end, the sheath lumen having a lumen diameter for receiving the dilator means inserted therein in an assembled position, and means for engaging the sheath distal end within the dilator outer surface at a junction of the sheath distal end with the dilator outer surface and providing a smooth transition for facilitating the introduction of the assembly of the dilator and introducer sheath into the body vessel lumen.

According to a second aspect, there is provided an introducer system comprising:
a dilator, the dilator having a distal end and a dilator outer surface, the dilator outer surface having a first diameter at a point spaced a first distance from the distal end, and a second diameter at a point spaced a second distance from the distal end, the second distance greater than the first distance and the second diameter less than the first diameter; and
an introducer sheath disposed over the dilator, the introducer sheath having a sheath lumen, the sheath lumen having a lumen diameter, the lumen diameter less than the first diameter, the introducer sheath further having means for permitting removal of the introducer sheath from a lead or catheter disposed therethrough without requiring the introducer sheath to be removed from an end of the lead or catheter disposed therethrough.

According to a third aspect, there is provided an introducer system for use with a catheter or lead comprising:
a sheath having a distal end and a second end, the sheath being compatible for insertion within a body, the sheath having a central lumen configured to permit introduction of at least one lead or catheter therethrough, the central lumen having a first diameter at the distal end and a second diameter at a point spaced apart from the distal end, the second diameter being greater than the first diameter;
means for permitting removal of the sheath from the lead or catheter disposed therethrough without requiring the sheath to be removed from an end of the lead or catheter disposed therethrough; and
a dilator having a first end and a second end, the first end of the dilator being tapered, the dilator disposed through the central lumen of the sheath.

The sheath preferably is constructed to readily tear in a longitudinal direction and thus permits the system to be removed from the venous system without having to withdraw the sheath over an end of the lead, etc.

The foregoing and other aspects of the present invention will be best appreciated with reference to the detailed description of preferred embodiments of the invention, given by way of example only, in conjunction with the accompanying drawings, wherein:
FIG. 1 depicts the venous positioning and placement of transvenous endocardial leads in a patient;
FIG. 2 depicts an appropriate entry site for implantation of a transvenous endocardial lead;
FIGS. 3 - 14 depict successive stages of introducing a transvenous endocardial lead into a vein;
FIG. 15 is a partial cross-section view of the transition zone of the dilator and surrounding introducer sheath in a distal end segment thereof depicting a first annular recess and sheath distal end opening shape in accordance with a first embodiment of the present invention;
FIG. 16 is a cross-section view of the transition zone of the dilator and surrounding introducer sheath in a distal end segment thereof depicting a second annular recess and sheath distal end opening shape in accordance with a second embodiment of the present invention; and
FIG. 17 is an enlarged, cross-section, detail view of a portion of the transition zone of the introducer sheath and dilator embodiment of FIG. 16.
FIG. 18 is a side view of an alternative embodiment of the present invention.
FIG. 19 is an enlarged, cross-section, detail view of a portion of the transition zone of the embodiment shown in FIG. 18.
FIG. 20 is an enlarged, cross-section, detail view of a portion of the transition zone of an alternative embodiment of the present invention.

The figures are not necessarily to scale.

It is to be understood that the present invention is not limited to use only in introducing atrial or ventricular pacing leads, and may be employed in introducing many of various types of therapeutic or diagnostic devices including transvenous leads intended to be disposed at various places within patient 10, including, for example, leads intended to be disposed within the patient's coronary sinus, as well as various other types of electrical leads, including nerve, muscle or defibrillation leads. It is to be further understood, moreover, the present invention may be employed in introducing many of various types of therapeutic or diagnostic catheters or probes and is not limited only to the introduction of electrical leads. For purposes of illustration only, however, embodiments of the present invention are described below in detail in the context of the introduction of endocardial pacing leads.

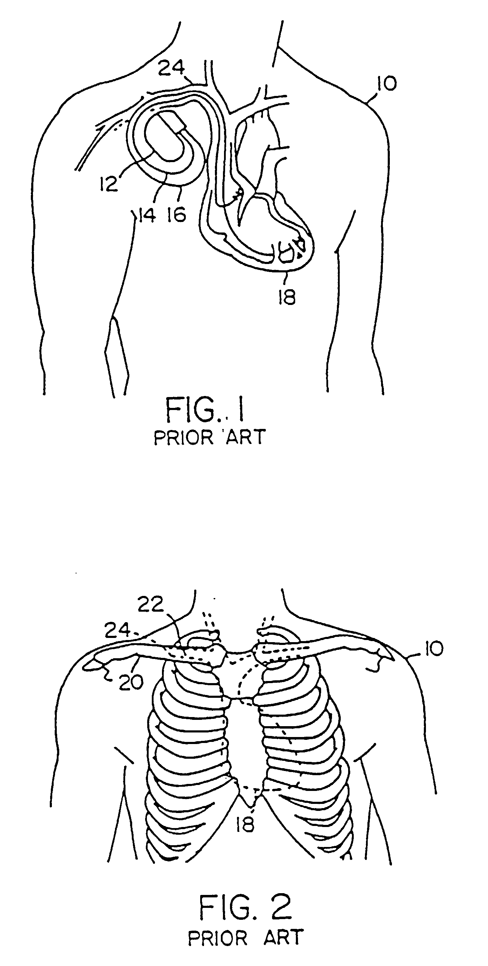

FIG. 1 depicts a typical arrangement of a pacing system implanted in a patient 10, the pacing system comprising a subcutaneously disposed pulse generator 12 and transvenous pacing leads 14 and 16. In FIG. 1, the distal end of pacing lead 14 is shown disposed generally in the atrial region of the patient's heart 18, while the distal end of pacing lead 16 is disposed generally in the ventricular region of heart 18.

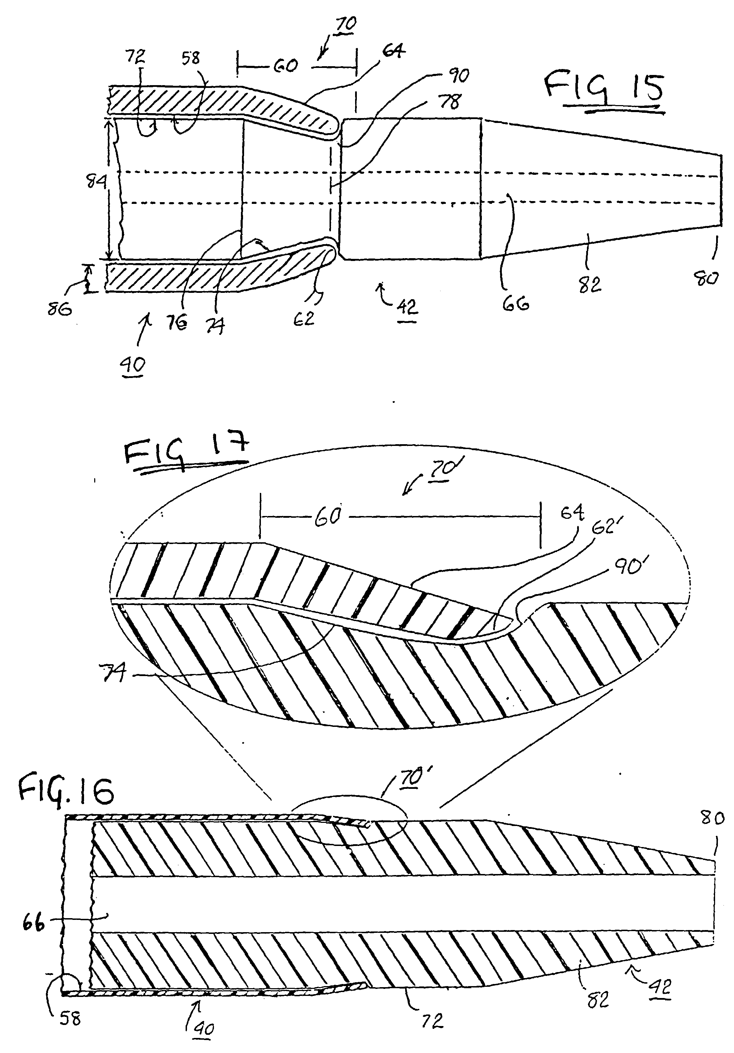

The preferred embodiments of the present invention are depicted in FIGS. 15 and 16. The method of lead introduction compatible with an introducer system in accordance with the present invention will be described with reference to the prior art procedure steps illustrated in FIGS. 2 through 14 referenced to the introduction of a pacing lead of FIG. 1 into a body vessel. The introducer system of the present invention may also be used in the introduction of catheters and probes into any similar body vessel, including blood vessels, using the steps illustrated in FIGS. 2 through 14.

Referring to FIG. 2, and in accordance with common practice in the medical arts, the entry site for a subclavian vein puncture is commonly chosen to be just below and slightly medial to the junction of the middle and inner third of the clavicle 20, at an area designated generally as 22 in FIG. 2. In FIG. 2, the patient's subclavian vein 24 and heart 18 are shown in phantom.

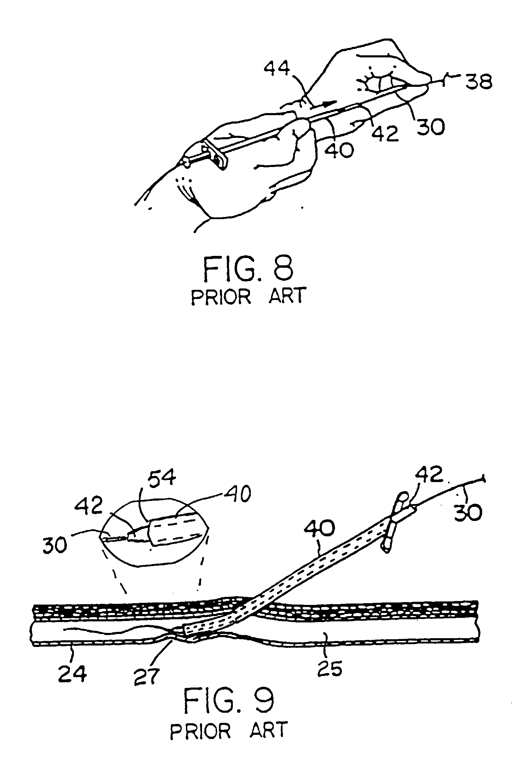

With respect to FIGS. 3 - 14, it should be understood that they are intended to illustrate the procedure in some detail but are not to scale. Specifically, the blood vessel or vein 24 is enlarged considerably relative to the apparatus that is described below from what is encountered at the subclavian puncture site and is typically encountered at any site in the body. The lumen of vein 24 is typically large enough to accommodate a needle and/or guide wire but must be enlarged to accommodate the sheath for allowing the eventual introduction of the lead, catheter or probe. Fortunately, while vein or other blood or body vessel walls are fragile, they are flexible and can typically be enlarged considerably. This enlargement of the vein lumen is not specifically shown in FIG. 9 - 14, but it should be understood that it does take place.

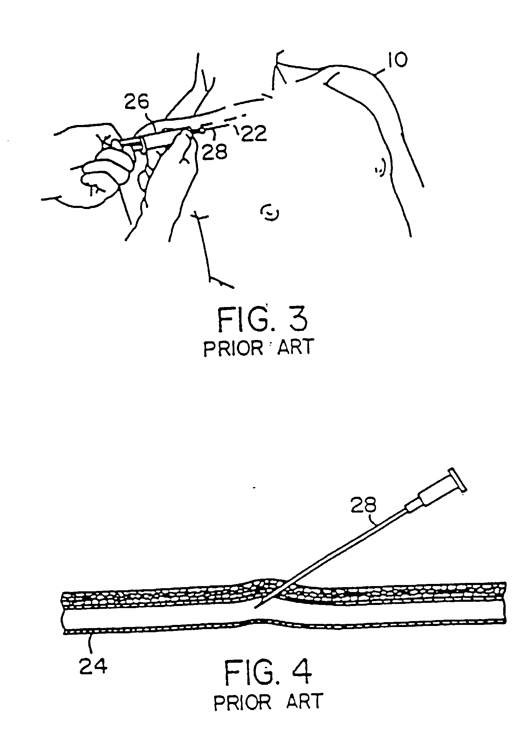

Turning to FIG. 3, the subclavian vein puncture is accomplished by the physician using a disposable syringe 26 having a thin-wall needle 28 detachably connected thereto. Aspiration is performed as the needle is advanced into the subclavian vein, to verify proper needle placement within vein 24. Next, aspirating syringe 26 is disconnected from needle 28, which remains in vein 24 as shown in FIG. 4. Typically, the physician will place his or her finger over the needle to avoid air aspiration and excessive bleeding.

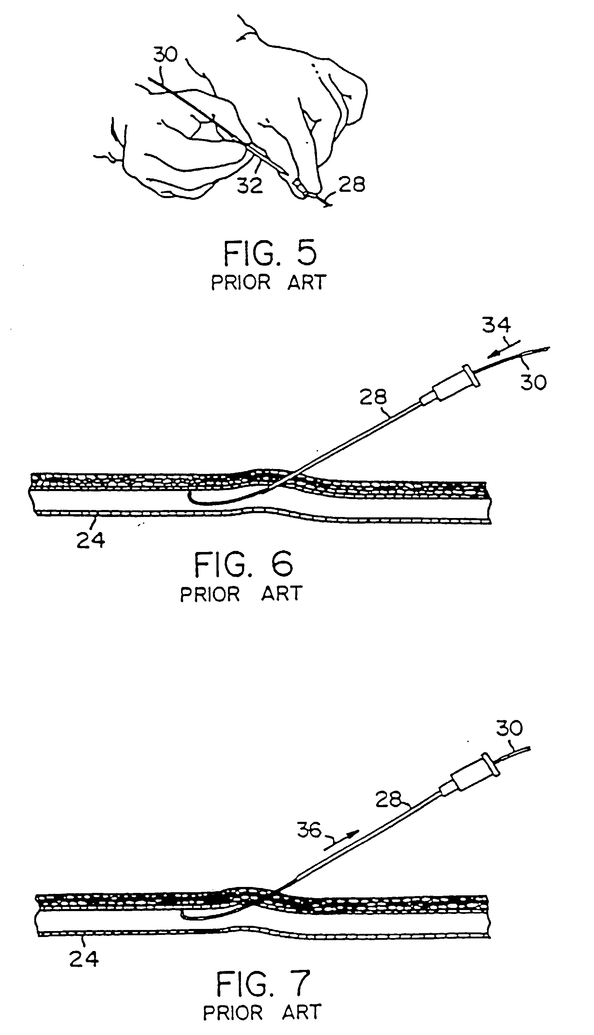

The next step in the lead implantation procedure involves insertion of a conventional J-type guide wire 30 through needle 28, as illustrated in FIG. 5. Typically, guide wire 30 is equipped with a tip deflector 32 for facilitating insertion of wire 30 into the lumen of needle 28. As shown in FIG. 6, as wire 30 is fed through needle 28 in the direction of arrow 34, the distal end of wire 30 exits the tip of needle 28, and wire 30 regains its "J" shape within vein 24. Once guide wire 30 has entered vein 24, needle 28 is withdrawn in the direction of arrow 36 in FIG. 7, leaving wire 30 in place. Guide wire 30 is advanced along vessel 24 until its distal end is disposed generally in the area of the patient's superior vena cava, leaving approximately 15 - 20 cm of the proximal end of guide wire 30 exposed.

A small skin incision 38 is made at the guide wire entry site, parallel to clavicle 20, as shown in FIG. 8. In the next stage of the implantation procedure, an introducer sheath 40 with vessel dilator 42, as an assembly, are threaded onto the proximal end of wire 30. Dilator 42 has a tapered distal end portion for dilating the lumen of body vessel or vein 24 on advancement of the distal end portion into the vein lumen. The dilator 42 has a cylindrical tubular body with a generally constant major diameter proximal to the tapered distal end portion enclosing a dilator lumen having proximal and distal end openings for receiving the guide wire so that the assembly can be advanced over it. The sheath 40 also has an elongate tubular body of relatively constant outside diameter and an inner sheath lumen allowing it to be fitted over the dilator 42 proximal to the tapered distal end portion. Sheath 40 and dilator 42 are advanced in the direction of arrow 44, through the subclavian fascia and into subclavian vein 24, until a short length (e.g., 2 - 8 cm) of sheath 40 and vessel dilator 42 remain exposed, as shown in FIG. 9.

In the prior art, the junction of the sheath 40 distal end over the dilator 42 outer surface presents a bull nose or annular step 54 which, however slight it may appear to be to the eye, can catch on the blood vessel wall during this advancement. As mentioned above, the distal end portion of the sheath 40 may also be tapered to diminish the height of the step 54, but some step height has to remain in order to preserve the integrity of the sheath 40 (i.e., to prevent it from tearing or rolling up) and to provide enough column strength to allow it to be manipulated in the vein lumen when the dilator 42 is removed.

Curves, bends, scar tissue or other irregularities form impediments 27 in the vein lumen 25 that can cause the step 54 to catch and impede progress, cause injury to the wall of the vein 24 and/or mis-align the introducer sheath lumen with the vein lumen 25. As mentioned above, the wall of vein 24 may have scar tissue from prior procedures forming impediments 27 that makes the introduction quite difficult.

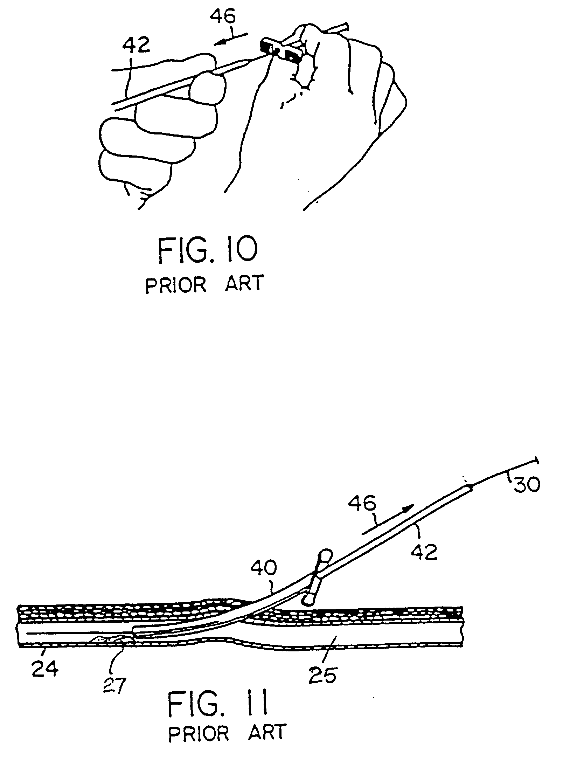

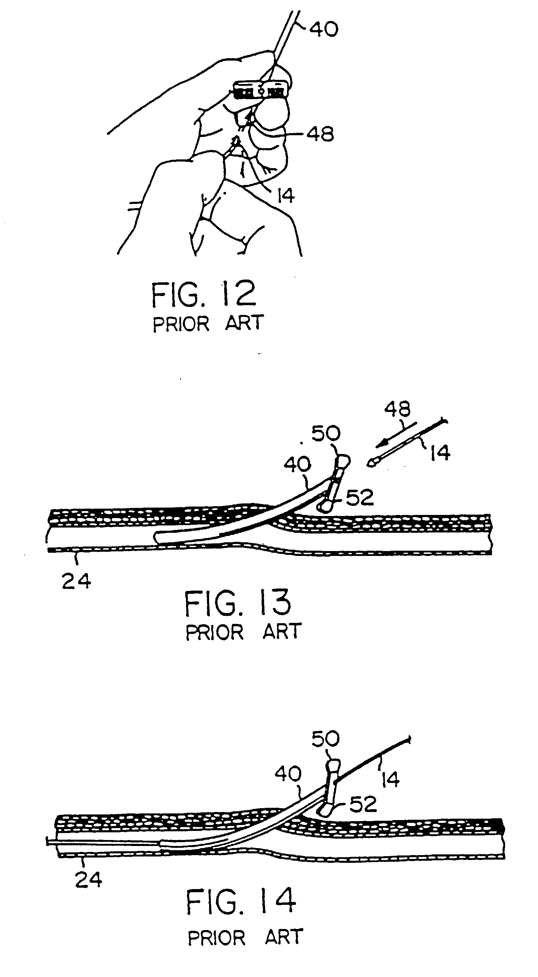

Next, as shown in FIGS. 10 and 11 (and assuming no such complications for purposes of completing the description of the procedure), vessel dilator 42 is withdrawn in the direction of arrow 46, and sheath 40 is introduced further within subclavian vein 24, leaving introducer sheath 40 and guide wire 30 in place with its distal end disposed within subclavian vein 24. Guide wire 30 may be removed at this point as well, although it may be left in place in case the lead needs to be repositioned or reinserted. As shown in FIG. 11, introducer sheath 40 must bend to conform to the shape of subclavian vein 24 to provide an unobstructed conduit for the pacing lead to be introduced. Through such curvature, moreover, the lead may be introduced so as to be aligned axially with vein 24 and not abut and damage the wall 25 of subclavian vein 24.

In the final stages of the lead implantation procedure, illustrated in FIGS. 12 - 14, pacing lead 14 is inserted into the proximal end of introducer sheath 40 in the direction of arrow 48, and advanced into the desired position within patient 10 through vein 24. Lastly, introducer sheath 40 is removed. Removal of introducer sheath 40 may be accomplished in one of several known ways, depending upon the particular type of introducer sheath 40. Preferably, sheath 40 includes means for permitting removal of sheath 40 from a lead disposed therethrough without requiring sheath 40 to be removed from an end of the lead.

In particular, introducer sheath 40 is split apart by grasping tabs 50 and 52 as it is being withdrawn from the lead introduction site. In the preferred embodiment linear scoring lines or other weakened line structures extend along the length of and within the wall of sheath 40 as shown in Boarini et al. U.S. Patent No. 4,411,654 and Vegoe et al U.S. Patent No. 5,180,372. Such weakened line structures may consist of material having the physical property of molecular orientation, whereby a tear in the material runs readily only in a longitudinal direction along the length of sheath 40. Whichever linear structure or modification of the sheath wall is employed, sheath 40 may be longitudinally split into two by grasping and pulling tabs 50 and 52 apart. Other sheaths are known which are severable by means of a special slitter device or the like, e.g. the above-referenced QUICKSPLIT® sheath.

As shown in FIG. 1, pacemaker pulse generator 12 may be coupled with two pacing leads 14 and 16 introduced percutaneously and transvenously in the manner described above. In that case, as with single-lead implants, it may be necessary to keep guide wire 30 in place until after the first lead has been implanted. Thus, as previously noted with reference to FIGS. 10 and 11, guide wire 30 may be left in place when dilator 42 is withdrawn. The first lead, if it is sufficiently small, may be introduced into subclavian vein 24 alongside guide wire 30. The first introducer sheath is then removed leaving guide wire 30 in place. A second assembled introducer sheath and vessel dilator can then be guided along guide wire 30 in the same manner as the first assembled sheath/dilator, before guide wire 30 is finally removed.

As mentioned above, the above described procedure may also be used in the introduction and placement of catheters or probes in blood vessels or other body vessels.

Turning now to the preferred embodiments of the invention, the dilator 42 and sheath 40 are configured to eliminate the annular step 54 and provide a smooth transition of the sheath distal end opening with the dilator outer surface when assembled together in the step of FIG. 9. FIG. 15 is an enlarged, partially cross-section view of the distal portion of the assembly of the introducer sheath 40 and dilator 42 used in the process of FIGS. 3 - 14 in accordance with a first embodiment of the invention. A distal portion of the dilator 42 is shown within the sheath lumen 58 of the distal end portion of the sheath 40, shown in cross-section. A distal transition zone 60 of the dilator 42 and the sheath 40 includes an annular recess 70 receiving a distal end band 64 and distal end 62 of the sheath 40 and shaped in accordance with a first embodiment of the present invention.

The proximal ends and major lengths of sheath 40 and dilator 42 may take any of the conventional configurations and are generally tubular bodies with each having a lumen and having relatively constant wall thicknesses and inner and outer diameters through their major lengths proximal to transition zone 60. As described above, the dilator lumen 66 (shown in phantom lines) receives the guide wire so that the assembly of the introducer sheath 40 and dilator 42 can be advanced distally over it. The introducer sheath lumen 58 fits over the major outer surface 72 of the dilator 42.

The elongate, tubular, dilator 42 has a distal tip end 80 of a relatively small diameter surrounding the distal end opening of the dilator lumen 66. A tapered distal end portion 82 extends proximally of the distal tip end 80 with a progressively increasing taper diameter from the distal tip end diameter to the dilator major diameter 84. The tapered distal end portion 82 is thereby formed for progressively dilating the body vessel or vein lumen diameter on advancement of the dilator distal end portion 82 through the body vessel or vein lumen.

The dilator 42 is formed with the annular recess 70 in its outer surface adjoining or somewhat proximal to the proximal terminus of the tapered distal end portion 82. However, the annular recess 70 may be formed immediately proximal to or within a proximal segment of the tapered distal end portion 82.

The elongate introducer sheath 40 is formed of a tubular body of relatively constant wall thickness 86 through its major length forming the sheath lumen 58 therein which extends between a distal opening in the sheath distal end 62 and a sheath proximal end opening (not shown in this figure). The sheath lumen 58 has a major lumen diameter of approximately the same dimension as the major diameter 84 of the dilator 42 for receiving the dilator 42 inserted therein in the depicted assembled position with the dilator distal end portion 82 extending distally of the sheath distal end 62. A slight separation is shown in FIG. 15 simply for convenience of illustrating the separate parts and may or may not be present in practice.

The sheath distal end 62 is formed with a reduced inner lumen diameter at the distal end opening thereof from the major inner diameter of the sheath lumen 58. The sheath distal end 62 may also have a distal end wall thickness that is reduced from the wall thickness 86 prevalent through the major length of the tubular body of the introducer sheath 40. When assembled as depicted in FIG. 15, sheath distal end 62 fits into the annular recess 70, so that the sheath distal end wall is submerged within the major outer diameter 84 of the dilator 42 (or the diameter of the tapered distal end portion 82 if the annular recess 70 is located within it). In this manner, a smooth transition zone 60 is provided for facilitating the introduction of the assembly of the dilator 42 and introducer sheath 40 into the body vessel as the body vessel is dilated by the tapered distal end portion 82 and the tapered exposed surface of the introducer sheath distal end portion 64.

The smooth transition zone 60 is further enhanced by forming the annular recess 70 to have a tapered recess band 74 having proximal and distal tapered band borders 76 and 78 defining a tapered band width therebetween. The tapered recess band 74 is formed extending proximally with a progressively increasing taper diameter from the reduced diameter of recess 70 at the distal tapered band border 78 to the major diameter 84 at the proximal tapered band border 76. This tapered recess band 74 accommodates the corresponding distal band 64 of the introducer sheath extending proximally from the distal end opening 62 and enhances the smooth transition zone 60. The sheath lumen 58 may also be formed to have a taper extending proximally from the distal tip opening 62 in the corresponding distal band 64 thereof.

Regardless of whether or not the sheath distal end band 64 and distal end 62 of the sheath 40 is tapered or narrowed, if the sheath 40 is formed of a stretchable material, and if the sheath lumen 58 inner diameter and the dilator major diameter 72 are interference dimensioned, the sheath distal end band 64 may contract into the recess 70 and bear against the tapered recess band 74.

As described above, it is necessary to remove the dilator 42 once full introduction of the sheath 40 has been achieved by retracting it proximally through the sheath lumen 58 while holding the sheath 40 steady. The annular recess 70 would inhibit that removal by engaging the sheath distal end 62. In the embodiment of FIG. 15, this concern is addressed by shaping the sheath distal end 62 and a distal, annular radiused recess band 90 of the annular recess 70 with radiused mating surfaces. The annular radiused band 90 distal to the tapered recess band 90 forms a sheath distal end 62 engaging surface to hold the sheath 40 in position on the dilator 42 during the advancement and manipulation of the assembly.

The radiused mating concave and convex surfaces of annular radiused recess band 90 and the sheath distal end 62, respectively, also provide a cam and follower action between them for engaging and expanding the sheath distal end 62 during retraction of the dilator 42. The cam and follower action expands the reduced lumen opening diameter of the distal end 62 as the convex shaped distal end opening 62 rides up or follows the concave radiused recess band 90 and onto the outer surface of diameter 72 of dilator 42 during the relative retraction of the dilator 42. In this manner the proximal retraction of the dilator 42 from the assembly with the introducer sheath 40 is effected as the annular radius cam surface tracks the annular radiused band surface to expand the distal end opening 62 reduced diameter to the major diameter 84 of the dilator 42.

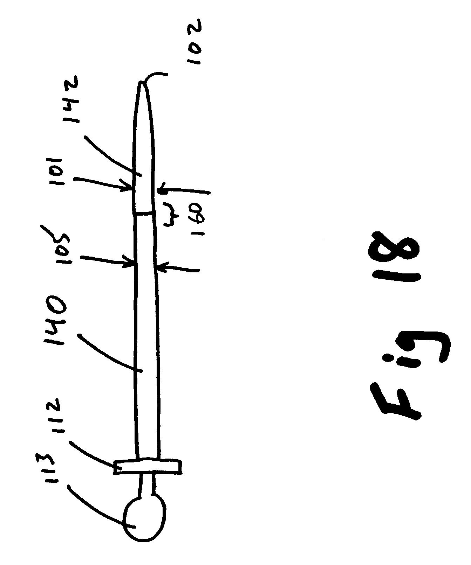

FIGS. 16 and 17 depict an alternative embodiment of a lead introducer system of the present invention. FIG. 16 is a cross-section view of the transition zone 60 of the dilator 42 and surrounding introducer sheath 40 in a distal end band 64 thereof depicting a second annular recess 70' and sheath distal end 62' shape in accordance with a second embodiment of the present invention. FIG. 17 is an enlarged, cross-section, detail view of a portion of the transition zone 60' of the introducer sheath and dilator embodiment of FIG. 16.

In this embodiment, the radiused recess band 90' is created with a larger radius relative to the band 74 so that it is less abrupt than the radiused recess band 90. The sheath distal end is also shaped with the corresponding larger radius that does not extend fully around the annular tip area. The resulting band 64 is tapered, and the distal tip 62 is recessed within the recess 70' below the adjacent outer surface major diameter 72 of dilator 42 but still provides a smooth transition zone 60.

Through the above described features of the preferred embodiments of the invention, the recession of the distal end 62 of the introducer sheath 40 within the annular recesses 70, 70' eliminates the annular step 54 of the prior art. In addition, it protects the relatively thin walled distal ends 62, 62' from handling damage or tearing that could itself impede advancement or cause injury to body vessel walls. A wide variety of configurations may be envisaged for the recesses 70, 70' and sheath distal tips 62, 62' that provide the smooth transition zone 60 as described above and allow the retraction of the dilator 42 from the sheath 40.

FIG. 18 depicts a still further alternative embodiment of the present invention. In this embodiment dilator 142 having handle 113 at proximal end is mated within introducer sheath 140 having pull tabs 112 such that the system has a constant-outer diameter across the transition zone 160, i.e. dilator diameter 101 is substantially equal to sheath outer diameter 105.

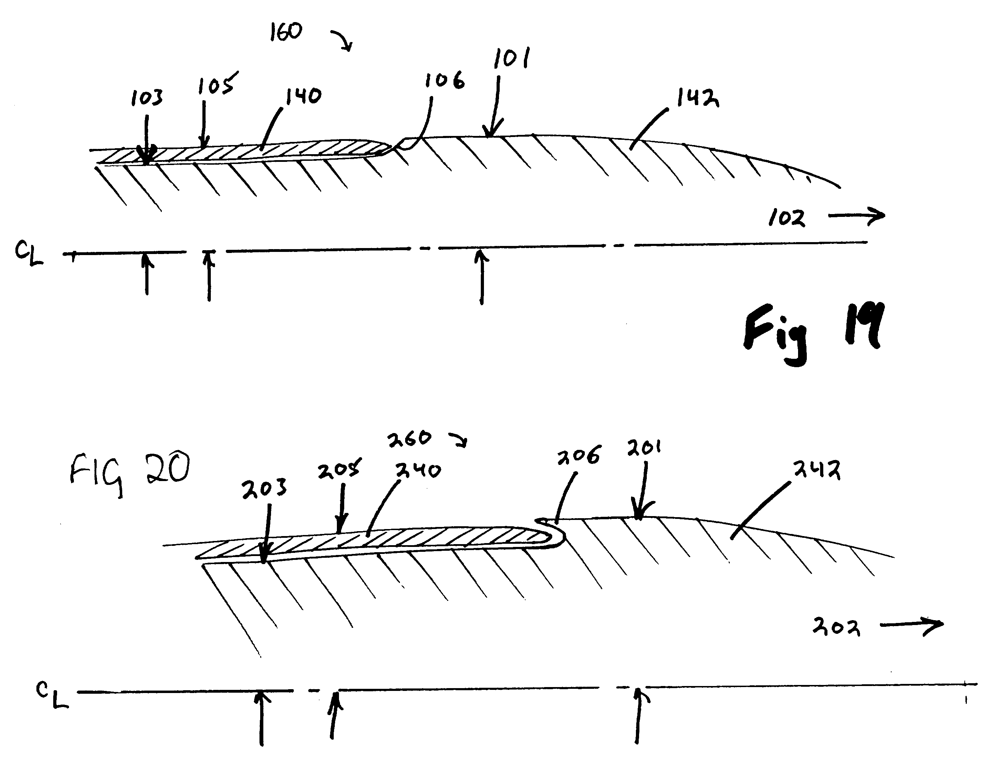

FIG. 19 is a detailed cross-section view of the transition zone 160 of dilator 142 and surrounding introducer sheath 140 depicted in FIG. 18. In this embodiment dilator 142 features a larger distal radius 101 towards the distal end 102 as compared to its proximal radius 103. Introducer sheath 140 is further dimensioned to have an outer radius 105 which is equal or less than the distal radius 101 of dilator 142. Dilator 142 joins sheath 140 at sloped section 106. Through such a configuration the total outer radius of the introducer sheath with dilator disposed therethrough is constant across the length of the transition zone 160. Thus the annular step 54 of the prior art introducer is eliminated and the system may be smoothly introduced.

FIG. 20 depicts a still further alternative embodiment of the present invention, and in particular a cross-section view of the transition zone 260 of dilator 242 and surrounding introducer sheath 240. In this embodiment dilator 242 features a larger distal radius 201 towards the distal end 202 as compared to its proximal radius 203. Introducer sheath 240 is further dimensioned to have an outer radius 205 which is equal or less than the distal radius 202 of dilator 242. Dilator 242 joins sheath 240 at annular overhang 206 so that the distal end of sheath 240 is disposed under the overhanging portion of annular overhang 206. Through such a configuration the total outer radius of the introducer sheath with dilator disposed therethrough is at most constant or may even taper a bit across the length of the transition zone 260 when compared from distal end to proximal end. Thus the annular step 54 of the prior art introducer is eliminated and the system may be smoothly introduced.

Although the invention has been described in detail with particular reference to a preferred embodiment and alternative embodiments thereof, it will be understood variations and modifications can be effected within the scope of the following claims.

## Claims

1. An introducer system for dilating a body vessel or the like for introducing an introducer sheath into the vessel lumen comprising:
elongate tubular dilator means (42) for dilating a body vessel lumen having a distal tip end (80) and a dilator outer surface (72) ;
elongate introducer sheath means (40) body enclosing a sheath lumen (58) therein and extending between openings in a sheath distal end (62) and a sheath proximal end, the sheath lumen (58) having a lumen diameter for receiving the dilator means inserted therein in an assembled position, and means for engaging the sheath distal end (62) within the dilator outer surface (72) at a junction of the sheath distal end (62) with the dilator outer surface (72) and providing a smooth transition (60) for facilitating the introduction of the assembly of the dilator means (42) and introducer sheath means (40) into the body vessel lumen.

2. The introducer system of claim 1 for dilating a body vessel to percutaneously position an introducer sheath (40) into the body vessel lumen wherein the dilator means (42) further comprises a dilator proximal end, and wherein the dilator outer surface (72) has a major diameter for dilating the body vessel lumen diameter on advancement of the dilator distal end portion through the body vessel lumen; and wherein the introducer sheath means (40) has a sheath distal end (62), a sheath proximal end, with the distal end portion extending distally of the sheath distal end in the assembled portion, and wherein:
the dilator means (42) is formed with an annular recess (74) in the dilator outer surface in proximity with the tapered distal end portion; and
the sheath distal end (64) is formed to fit into the annular recess, whereby the junction of the sheath distal end with the dilator outer surface provides a smooth transition for facilitating the introduction of the assembly of the dilator and introducer sheath into the body vessel and for dilating the body vessel lumen as the assembly is advanced within the body vessel lumen.

3. The introducer system of claim 2 wherein:
the elongate tubular dilator means (42) has a tapered distal end portion extending proximally of the distal tip end and the dilator outer surface major diameter extends between the dilator proximal end and the tapered distal end portion, the tapered distal end portion formed with a progressively increasing taper diameter from the distal tip end to the dilator major diameter for dilating the body vessel lumen diameter on advancement of the dilator distal end portion through the body vessel lumen; and
the annular recess (74) is formed proximally with respect to the tapered distal end portion of the dilator.

4. The introducer system of claim 2 or 3 wherein the annular recess (70) is formed having a tapered recess band having a tapered band width and extending proximally with a progressively increasing taper diameter from the reduced diameter to the major diameter for accommodating a distal band of the introducer sheath means adjacent to the sheath distal end.

5. The introducer system of claim 4 wherein the annular recess is formed having an annular radiused band distal to the tapered recess band (74) forming a sheath distal tip engaging and expanding surface.

6. The introducer system of claim 5 wherein the distal tip of the introducer sheath means is formed with an annular radius follower surface shape substantially corresponding to the shape of the annular radiused recess band, whereby proximal retraction of the dilator means from the assembly with the introducer sheath means is effected as the annular radius follower surface tracks the annular radiused band surface to expand the distal lumen opening in the sheath distal end over the dilator means.

7. The introducer system of claim 2 wherein the annular recess is formed having a tapered recess band (74) having proximal and distal tapered band borders and a tapered band width and formed extending proximally with a progressively increasing taper diameter from the reduced diameter at the distal tapered band border to the major diameter at the proximal tapered band border for accommodating a distal band of the introducer sheath adjacent to the sheath distal end.

8. The introducer system of claim 7 wherein the annular recess is formed having an annular radiused band having a proximal radius band border substantially coincident with the distal tapered band border and a distal radius band border at the juncture of the annular radiused recess band with the dilator outer surface.

9. The introducer system of claim 8 wherein the distal tip of the introducer sheath means is formed with an annular radius surface substantially corresponding to the annular radiused recess band, whereby proximal retraction of the dilator means from the assembly with the introducer sheath means is effected as the annular radius surface tracks the annular radiused band surface to expand the distal lumen opening in the sheath distal end over the dilator means.

10. The introducer system of claim 9 wherein:
the distal tip annular radiused surface is a convex surface; and
the annular radiused recess band is formed with a concave surface.

11. The introducer system of any preceding claim further comprising means for releasing the sheath distal end from within the dilator outer surface and for allowing proximal retraction of the dilator means from the sheath lumen.

12. An introducer system comprising:
a dilator (42), the dilator having a distal end and a dilator outer surface, the dilator outer surface having a first diameter at a point spaced a first distance from the distal end, and a second diameter at a point spaced a second distance from the distal end, the second distance greater than the first distance and the second diameter less than the first diameter; and
an introducer sheath (40) disposed over the dilator (42), the introducer sheath (40) having a sheath lumen (58), the sheath lumen having a lumen diameter, the lumen diameter less than the first diameter, the introducer sheath further having means for permitting removal of the introducer sheath from a lead or catheter disposed therethrough without requiring the introducer sheath to be removed from an end of the lead or catheter disposed therethrough.

13. The introducer system of claim 12 wherein the dilator is formed with an annular recess in the dilator outer surface in proximity with the tapered distal end portion.

14. The introducer system of claim 12 wherein the dilator is formed with an annular overhang in the dilator outer surface in proximity with the tapered distal end portion.

15. The introducer system of claim 12 wherein the dilator has a tapered distal end portion extending proximally of the distal tip end and the dilator outer surface major diameter extends between the dilator proximal end and the tapered distal end portion, the tapered distal end portion formed with a progressively increasing taper diameter from the distal tip end to the dilator major diameter for dilating the body vessel lumen diameter on advancement of the dilator distal end portion through the body vessel lumen; and
the annular recess is formed proximally with respect to the tapered distal end portion of the dilator.

16. An introducer system for use with a catheter or lead comprising:
a sheath (40) having a distal end and a second end, the sheath being compatible for insertion within a body, the sheath having a central lumen (58) configured to permit introduction of at least one lead or catheter therethrough, the central lumen having a first diameter at the distal end and a second diameter at a point spaced apart from the distal end, the second diameter being greater than the first diameter;
means for permitting removal of the sheath from the lead or catheter disposed therethrough without requiring the sheath to be removed from an end of the lead or catheter disposed therethrough; and
a dilator (42) having a first end and a second end, the first end of the dilator being tapered, the dilator disposed through the central lumen of the sheath.

17. The introducer system of claim 16 wherein the means for permitting removal of the sheath comprises means for splitting the sheath away from the lead or catheter disposed therethrough.

18. The introducer system of claim 17 wherein the means for splitting the sheath comprise a first tab on a first side of the second end of the sheath, and a second tab on a second side of the second end of the sheath whereby, when the tabs are pulled apart, the sheath tears longitudinally separating the sheath from the lead or catheter disposed therethrough.

19. The introducer system of claim 17 wherein the means for splitting the sheath comprises a weakened section extending longitudinally from the first end to the second end of the sheath.

20. The introducer system of claim 19 wherein the weakened section comprises a score line.

21. The introducer system of claim 19 wherein the weakened section comprises a section of material having the physical property of molecular orientation whereby a tear in the material runs readily only in a longitudinal direction along the length of the sheath.
